# EUROPEAN PATENT APPLICATION

(11) **EP 3 351 221 A1**
(43) Date of publication of application: **25.07.2018**
(21) Application number: 16875144.4
(22) Date of filing: 22.07.2016
(51) Int. Cl.: A61F 13/47, A61F 13/476, A61F 13/56

(54) **ABSORBENT ARTICLE**

(30) Priority: 16.12.2015 JP 2015245042
(71) Applicant: Unicharm Corporation, Ehime 799-0111 (JP)
(72) Inventor: AKIYAMA, Saeko, Kanonji-shi Kagawa 769-1602 (JP); KUDO, Jun, Kanonji-shi Kagawa 769-1602 (JP); TERASOMA, Nozomi, Kanonji-shi Kagawa 769-1602 (JP)
(74) Representative: Dolleymores
(86) International application number: PCT/JP2016/071625
(87) International publication number: WO 2017/104157

(57) **Abstract**

An absorbent article including: an absorbent body (3) ; a top sheet (2) ; a back sheet (4) ; a pair of wings (100) provided at a center portion in the longitudinal direction and projecting outwardly in the width direction; a wing adhesive portion (52) provided on the non-skin side of each of the wings (100) ; and a body adhesive portion (51) provided on the non-skin side of the back sheet (4) along the longitudinal direction and being different from the wing adhesive portion (52); a bending guide portion (30) that guides the absorbent body (3) to be bent to the skin side being provided along the longitudinal direction at a center portion in the width direction, the bending guide portion (30) including an overlapping portion overlapping with the wing adhesive portion (52) in the longitudinal direction, a divided region (511, 512) that divides the body adhesive portion (51) into front and back being provided in a front side or back side of the wing adhesive portion (52) in the longitudinal direction.

## Description

### [Technical Field]

The present disclosure relates to absorbent articles.

### [Background Art]

Sanitary napkins are conventionally known as absorbent articles. In PTL 1, for example, there is disclosed a sanitary napkin including a liquid-permeable top surface sheet, a liquid-impermeable back surface sheet, and an absorbent body interposed between these two sheets.

This sanitary napkin includes an embossed groove along a longitudinal direction on a non-skin-contacting side of an absorbent body and at a center part in a width direction. When the sanitary napkin is worn, a central region of the absorbent body is deformed so as to be uplifted to a skin side of a wearer due to this embossed groove, thereby being in close contact with a region (blood draining port) contacting a discharge opening. Adhesive portions (shift-prevention adhesive agent layers) for fixing the napkin to an undergarment are continuously provided along the longitudinal direction on both sides of the embossed groove in the width direction.

### [Citation List]

### [Patent Literature]

[PTL 1] Japanese Unexamined Patent Application Publication No. 2012-95685

### [Summary of Invention]

### [Technical Problem]

In an absorbent article that is deformed to have a protruded shape so that a center portion in the width direction protrudes toward a skin side of a wearer when the napkin is worn, as in a sanitary napkin described in PTL 1, when adhesive portions are continuously provided from the front side to back side along the longitudinal direction, it is difficult to raise a napkin 1 to the skin side of the wearer with a region crossing the adhesive portions serving as a starting point, due to stiffness of the adhesive portions. Accordingly, it becomes difficult to raise the napkin 1 toward the skin side of the wearer while maintaining a flat shape in the front side or back side of the region contacting the discharge opening and to allow the napkin 1 to be in flat contact with the skin of the wearer, thus deteriorating a fitting property.

The present invention has been made in view of the above circumstances and an objective thereof is to provide an absorbent article capable of improving a fitting property even in the front side or back side of the region contacting the discharge opening.

### [Solution to Problem]

A principal aspect of the present invention to achieve the above advantage is an absorbent article including a longitudinal direction, a width direction and a thickness direction, the absorbent article including: an absorbent body that absorbs liquid; a skin side sheet disposed on a skin side of the absorbent body in the thickness direction; a non-skin side sheet disposed on a non-skin side of the absorbent body in the thickness direction; a pair of wings provided at a center portion in the longitudinal direction and projecting outwardly in the width direction; a wing adhesive portion provided on the non-skin side of each of the wings; and a body adhesive portion provided on the non-skin side of the non-skin side sheet along the longitudinal direction, the body adhesive portion being different from the wing adhesive portion; a bending guide portion that guides the absorbent body to be bent to the skin side being provided along the longitudinal direction at a center portion in the width direction, the bending guide portion including an overlapping portion overlapping with the wing adhesive portion in the longitudinal direction, a divided region that divides the body adhesive portion into front and back being provided in a front side or back side of the wing adhesive portion in the longitudinal direction. Other features of the present invention will be made clear through the present specification with reference to the accompanying drawings.

### [Advantageous Effects of Invention]

According to the present disclosure, it is possible to improve a fitting property even in the front side or back side of the region contacting the discharge opening.

### [Brief Description of Drawings]

[FIG. 1] FIG. 1 is a schematic plan view of a napkin according to an embodiment of the present disclosure when seen from the skin side in the thickness direction.
[FIG. 2] FIG. 2 is a schematic plan view of the napkin when seen from the non-skin side in the thickness direction.
[FIG. 3] FIG. 3 is a cross-sectional view taken along line III - III of FIG. 1.
[FIG. 4] FIG. 4A is a schematic cross-sectional view illustrating a use aspect of the napkin and a state of the napkin when being worn, and FIG. 4B is a schematic perspective view illustrating an overall shape of the napkin when being worn.
[FIG. 5] FIG. 5 is an explanatory view illustrating a configuration and a positional relationship of compressed portions.
[FIG. 6] FIG. 6 is an enlarged view of an A-portion of FIG. 5.

### [Description of Embodiments]

At least the following matters will be made clear by the description in the present specification and the accompanying drawings.

Disclosed is an absorbent article including a longitudinal direction, a width direction and a thickness direction, the absorbent article including: an absorbent body that absorbs liquid; a skin side sheet disposed on a skin side of the absorbent body in the thickness direction; a non-skin side sheet disposed on a non-skin side of the absorbent body in the thickness direction; a pair of wings provided at a center portion in the longitudinal direction and projecting outwardly in the width direction; a wing adhesive portion provided on the non-skin side of each of the wings; and a body adhesive portion provided on the non-skin side of the non-skin side sheet along the longitudinal direction, the body adhesive portion being different from the wing adhesive portion; a bending guide portion that guides the absorbent body to be bent to the skin side being provided along the longitudinal direction at a center portion in the width direction, the bending guide portion including an overlapping portion overlapping with the wing adhesive portion in the longitudinal direction, a divided region that divides the body adhesive portion into front and back being provided in a front side or back side of the wing adhesive portion in the longitudinal direction.

According to such an absorbent article, since the body adhesive portion is divided at a position in the front side or back side of the wing adhesive portions in the longitudinal direction, the center portion in the width direction of the region contacting the discharge opening (a region overlapping with the wing adhesive portions in the longitudinal direction, that is, a region sandwiched between a pair of wing adhesive portions in the width direction) can be easily deformed into a protruded shape toward the skin side by the bending guide portion, and thus the guiding for the protruded deformation by the bending guide portion is eased in the front side or back side of the region contacting the discharge opening, thereby allowing the flat shape to be easily maintained. Consequently, the absorbent article can be easily in flat contact with the skin of the wearer in the front side or back side of the region contacting the discharge opening, thereby improving the fitting property.

In this absorbent article, it is preferable that the wings are provided in a region in the longitudinal direction between a position where a length in the width direction is the shortest in the front side of the wing adhesive portion and a position where a length in the width direction is the shortest in the back side of the wing adhesive portion, and the divided region is provided in a front side of a front end of each of the wings or in a back side of a back end of each of the wings in the longitudinal direction.

According to such an absorbent article, the divided region is provided in the further front side of the front end of the wings or in the further back side of the back end of the wings, and thus the protruded shape by the bending guide portion can be maintained to improve the fitting property with respect to the discharge opening in wider areas positioned at the crotch of the wearer.

In this absorbent article, it is preferable that the divided region is provided in the front side of the front end of each of the wings and is also provided in the back side of the back end of each of the wings in the longitudinal direction.

According to such an absorbent article, since the body adhesive portion is divided in the front side and back side of the wings in the longitudinal direction, the guiding for the protruded deformation by the bending guide portion is eased even in the both front side and back side, thereby allowing the flat shape to be easily maintained. Consequently, the absorbent article can be easily in flat contact with the skin of the wearer in the front side or back side of the region contacting the discharge opening. Thus, the fitting property is further improved.

In this absorbent article, it is preferable that a folding line along the width direction is positioned in the divided region.

According to such an absorbent article, the occurrence of wrinkles and distortion due to the folding line can be suppressed in the front side or back side of the divided region by disposing the folding line for an individual package in the divided region, and the guiding for the protruded deformation by the bending guide portion can be cut off, thereby allowing the flat shape to be easily maintained.

In this absorbent article, it is preferable that the bending guide portion and the body adhesive portion do not include an overlapping portion in the width direction.

According to such an absorbent article, since the body adhesive portions are not each disposed at a position where the bending guide portion is provided, when the absorbent article is deformed into a protruded shape toward the skin side of the wearer, the situation that the body adhesive portions approach with one another in the width direction and are bonded is suppressed. Thus, the protruded shape by the bending guide portion is neatly and easily formed in the region contacting the discharge opening.

In this absorbent article, it is preferable that compressed portions along the longitudinal direction are provided on both sides in the width direction of the bending guide portion, and
the compressed portions are divided into front and back in the divided region.

According to such an absorbent article, the compressed portions are also divided together with the body adhesive portions in the divided region, and thus the guiding for the protruded deformation by the bending guide portion is further eased in the front side of back side of the region contacting the discharge opening, thereby allowing the flat shape to be easily maintained.

In this absorbent article, it is preferable that tip portions at a side of the divided region of central compressed portions positioned on a side of the center portion in the longitudinal direction in the compressed portions are tilted from an inside to an outside in the width direction.

According to such an absorbent article, the tip portions of the central compressed portions are tilted from the inside to the outside in the width direction, and thus the region sandwiched between the compressed portions is widened from the center side toward the front side or from the center side toward the back side, thereby allowing the absorbent article to be easily guided into the flat shape toward the front side or the back side.

In this absorbent article, it is preferable that the tip portions at a side of the divided region of the central compressed portions each include, in the longitudinal direction, an overlapping portion overlapping with a tip portion at a side of the divided region of a front or back compressed portion positioned in a front side or back side of the central compressed portions in the longitudinal direction.

According to such an absorbent article, since the region sandwiched between each of the tip portions of the central compressed portions and the tip portion of the front or back compressed portion is sandwiched by the compressed portions (the central compressed portions and the front or back compressed portion) having high stiffness, wrinkles and distortion are less likely to occur in the region, and the flat shape can be easily maintained. Accordingly, the raising of the absorbent article toward the skin side of the wearer can be guided in a more flat manner in the front side or back side of the region contacting the discharge opening.

In this absorbent article, it is preferable that a folding line along the width direction is positioned at a center in the longitudinal direction of the divided region, and a region interposed between each of the tip portions of the central compressed portions and the tip portion of the front or back compressed portion include a non-overlapping portion that does not overlap with the folding line.

According to such an absorbent article, the region sandwiched between each of the tip portions of the central compressed portions and the tip portion of the front or back compressed portion include a non-overlapping portion that does not overlap with the folding line, and thus the flat shape of the region can be maintained in the non-overlapping portion. Consequently, the raising of the absorbent article toward the skin side of the wearer can be guided in a more flat manner in the front side or back side of the region contacting the discharge opening.

In this absorbent article, it is preferable that the tip portion of the front or back compressed portion is positioned inwardly in the width direction with respect to each of the tip portions of the central compressed portions.

According to such an absorbent article, since the tip portion of the front or back compressed portion is positioned inwardly in the width direction with respect to each of the tip portions of the central compressed portions, the force guiding the absorbent article into the protruded shape by the bending guide portion more easily escapes toward the outside in the width direction. Accordingly, the flat shape can be easily made in the front side or back side of the region contacting the discharge opening.

In this absorbent article, it is preferable that a crossing compressed portion that crosses the center portion in the width direction is provided in a back side of the wing adhesive portion in the longitudinal direction.

According to such an absorbent article, the guiding for the protruded deformation by the bending guide portion provided in the center portion in the width direction is less likely transmitted in the back side of the region contacting the discharge opening, thereby allowing the flat shape to be easily maintained in the back side.

In this absorbent article, it is preferable that the bending guide portion is provided at a position overlapping with the wings in the longitudinal direction.

According to such an absorbent article, since the bending guide portion is provided at a position (at the center portion in the longitudinal direction) overlapping with the wings in the longitudinal direction, an effect of the protruded deformation by the bending guide portion is suppressed in the front side or back side of the region in the width direction between the pair of wings including the region contacting the discharge opening, thereby allowing the flat shape to be more easily maintained.

=== Embodiments === A sanitary napkin 1 (hereinafter, merely referred to as a napkin 1) will be described as an example of an absorbent article associated with an embodiment of the present disclosure. The absorbent article also includes an absorbent sheet for daily vaginal discharge (e.g. a panty liner) or the like, and thus is not limited to a sanitary napkin.

### <Configuration of Napkin 1>

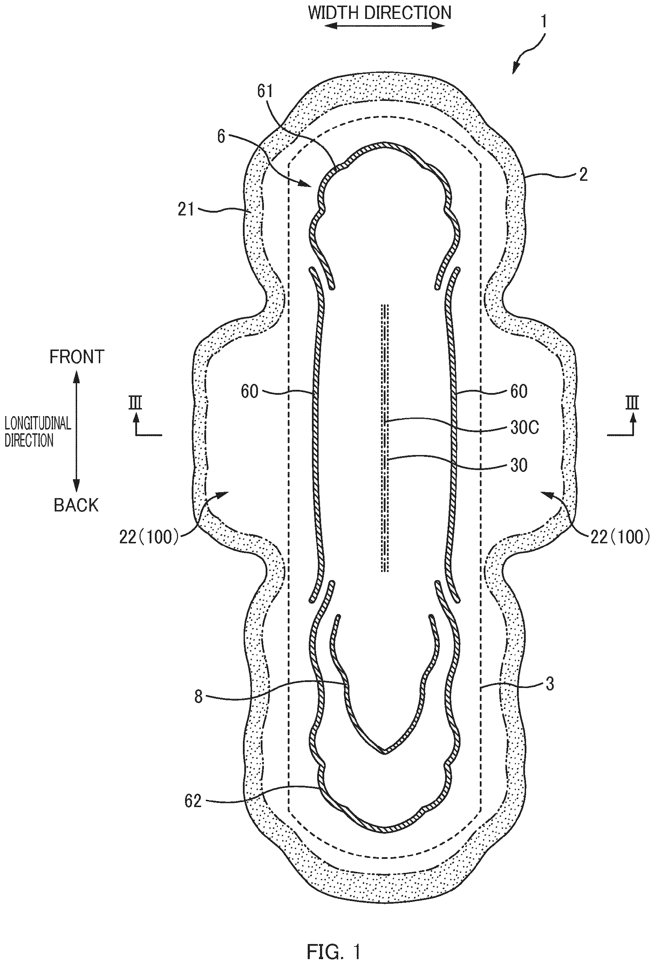

First, the configuration of the napkin 1 will be described with reference to FIGs. 1 to 3.

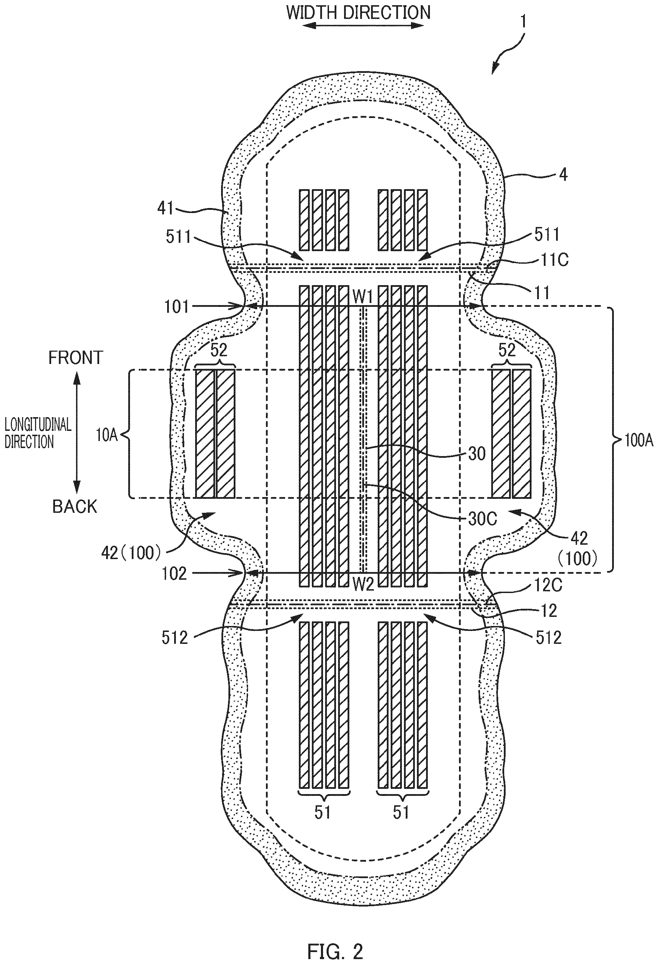

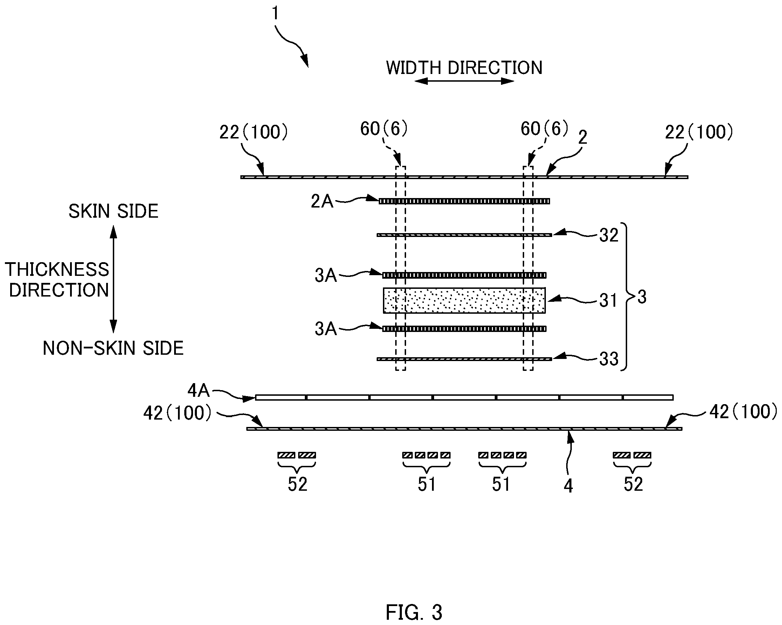

FIG. 1 is a schematic plan view of the napkin 1 associated with an embodiment of the present disclosure when seen from a skin side in the thickness direction. FIG. 2 is a schematic plan view of the napkin 1 when seen from a non-skin side in the thickness direction. FIG. 3 is a cross-sectional view taken along line III - III of FIG. 1.

As illustrated in FIG. 1 and FIG. 2, the napkin 1 is a sheet-shaped member having a longitudinally-elongated shape when seen in a planar view, and also includes a longitudinal direction, a width direction and a thickness direction. In the following descriptions, the "longitudinal direction" indicates a longitudinal direction of a product which is the napkin 1, the "thickness direction" indicates a direction of thickness of a product which is the napkin 1, and the "the width direction" indicates a lateral direction orthogonal to the longitudinal direction and the thickness direction. Note that, a direction of the front side of a user is referred to as a "front", and a direction of the back side of the user is referred to as a "back" in the longitudinal direction when the napkin 1 is used. Further, the thickness direction includes a "skin side" that is to be a side in contact with the skin of the user when the napkin 1 is used and a "non-skin side" opposite thereto.

As illustrated in Fig. 3, the napkin 1 includes an absorbent body 3 that absorbs liquid such as menstrual blood, a top sheet 2 disposed on the skin side of the absorbent body 3 in the thickness direction, and a back sheet 4 disposed on the non-skin side of the absorbent body 3 in the thickness direction.

The napkin 1 includes a pair of wings 100 that is provided at a center portion in the longitudinal direction and projects outwardly in the width direction. Wing adhesive portions 52 are provided on the non-skin side of each of the wings 100. When the napkin 1 is used, each wing 100 is folded toward the non-skin side, and each wing adhesive portion 52 is attached to an outer face of the crotch portion such as an undergarment to fix the napkin 1 to the undergarment or the like.

Here, the center portion in the width direction in a region sandwiched between one wing adhesive portion 52 and another wing adhesive portion 52 in the width direction is a portion with which the discharge opening of the wearer comes into contact when the napkin 1 is worn. In the following description, a region that is sandwiched between the one wing adhesive portion 52 and the other wing adhesive portion 52 in the width direction and is from a front end to a back end of each of the wing adhesive portions 52 in the longitudinal direction is referred as "a discharge opening contact region 10A" (see FIG. 2).

As illustrated in FIG. 2, the wings 100 are provided in a region in the longitudinal direction between a position in which the length in the width direction in the front side of the wing adhesive portions 52 is the shortest (W1 in FIG. 2) and a position in which the length in the width direction in the back side of the wing adhesive portions 52 is the shortest (W2 in FIG. 2). In the following description, the region in the longitudinal direction in which these wings 100 are provided is referred to as "a wing region 100A". This wing region 100A is a region extending to the front side and back side of the discharge opening contact region 10A in the longitudinal direction.

As illustrated in FIG. 3, the top sheet 2 and the absorbent body 3 are bonded in the thickness direction with a front surface adhesive 2A, and the absorbent body 3 and the back sheet 4 are bonded in the thickness direction with a back surface adhesive 4A. As the front surface adhesive 2A and the back surface adhesive 4A, for example, a hot-melt adhesive or the like can be used, and as an application pattern of the front surface adhesive 2A and the back surface adhesive 4A, for example, a Ω pattern, a spiral pattern, a stripe pattern or the like can be exemplified.

The top sheet 2 is an aspect of a permeable skin side sheet that allows liquid such as menstrual blood to permeate from the skin side to the non-skin side in the thickness direction, and a soft sheet member such as air through nonwoven fabric can be used. The top sheet 2 is formed larger than the absorbent body 3 when seen in a planar view as illustrated in FIG. 1 and covers the absorbent body 3 from the skin side in the thickness direction as illustrated in FIG. 3.

The top sheet 2 includes: an outer peripheral portion 21 (shown by a dotted pattern in FIG. 1) positioned outside the absorbent body 3 in the longitudinal direction and the width direction; and a pair of projecting portions 22 projecting outwardly in the width direction at the center portion in the longitudinal direction. In FIG. 1, the absorbent body 3 is illustrated by broken lines, and an inner edge of the outer peripheral portion 21 is illustrated by double-dotted chained lines, respectively.

The absorbent body 3 is a member that absorbs liquid such as menstrual blood and holds it inside. As illustrated in FIG. 3, the absorbent body 3 includes: an absorbent core 31; a skin side cover sheet 32 disposed on the skin side (top sheet 2 side) of the absorbent core 31 in the thickness direction; and a non-skin side cover sheet 33 disposed on the non-skin side (back sheet 4 side) of the absorbent core 31 in the thickness direction.

As illustrated in FIG. 3, the absorbent core 31 and the skin side cover sheet 32, and the absorbent core 31 and the non-skin side cover sheet 33 are respectively bonded in the thickness direction with an absorbent body adhesive 3A such as a hot-melt adhesive.

For the absorbent core 31, for example, liquid absorbent fiber such as pulp fiber is used. Note that, in addition to the liquid absorbent fiber, super absorbent polymer (so-called SAP) or the like may be contained. For the skin side cover sheet 32, for example, a member having an excellent liquid permeability such as air through nonwoven fabric is used. For the non-skin side cover sheet 33, for example, a member having an excellent flexibility such as SMS (spunbond/melted-blown/spunbond) nonwoven fabric is used.

In the absorbent body 3, a bending guide portion 30 that guides the absorbent body 3 so as to be bent to the skin side is provided along the longitudinal direction at the center portion in the width direction. Here, more specifically, the state of "being bent to the skin side in the thickness direction" refers to a state of being bent by protruding to the skin side to be deformed in a protruded shape.

In the present embodiment, the bending guide portion 30 is provided at a position overlapping with each wing 100 in the longitudinal direction, that is, in the wing region 100A. In this way, even when the bending guide portion 30 is provided only in a part in the longitudinal direction, the napkin 1 more easily protrudes to the skin side to be deformed in a protruded shape, compared with a case in which the bending guide portion 30 is not provided.

Note that, the bending guide portion 30 may include an overlapping portion overlapping with the wing adhesive portions 52 at least in the longitudinal direction, that is, may be provided in the discharge opening contact region 10A, and may also be provided over the entire longitudinal direction of the absorbent body 3.

This bending guide portion 30 may be formed by folding the absorbent body 3 in advance at a manufacturing step, and also may be formed by embossing, a slit-shaped groove (hinge) or the like, for example. In the present embodiment, the bending guide portion 30 is formed in the absorbent body 3. However, the invention is not limited thereto, and the bending guide portion 30 may be formed, for example, by folding the whole napkin 1 in advance.

As illustrated in FIG. 1 and FIG. 2, the bending guide portion 30 has a shape including a predetermined width with a reference line 30C as the center in the width direction. In FIG. 1 and FIG. 2, the reference line 30C is illustrated by single-dotted chain lines, and both ends of the bending guide portion 30 in the width direction are illustrated by broken lines, respectively.

The back sheet 4 is an aspect of a liquid impermeable non-skin side sheet having a leak prevention function, and for example, a resin film such as polyethylene (PE) can be used. As illustrated in FIG. 2, similar to the top sheet 2, the back sheet 4 is formed larger than the absorbent body 3 when seen in a planar view, and covers the absorbent body 3 from the non-skin side in the thickness direction as illustrated in FIG. 3.

The back sheet 4 includes: an outer peripheral portion 41 (shown by a dotted pattern in FIG. 2) positioned outside the absorbent body 3 in the longitudinal direction and the width direction; and a pair of projecting portions 42 projecting outwardly in the width direction at a center portion in the longitudinal direction. In FIG. 2, the absorbent body 3 is illustrated by broken lines, and an inner edge of the outer peripheral portion 41 is illustrated by double-dotted chained lines, respectively.

The napkin 1 is configured so that the outer peripheral portion 21 (see FIG. 1) of the top sheet 2 and the outer peripheral portion 41 (see FIG. 2) of the back sheet 4 are joined to hold the absorbent body 3 between the top sheet 2 and the back sheet 4. Moreover, a pair of wings 100 is formed by joining a pair of projecting portions 32 (see FIG. 1) of the top sheet 3 and a pair of projecting portions 42 (see FIG. 1) of the back sheet 4.

The wing adhesive portions 52 and body adhesive portions 51 different from the wing adhesive portions 52 are provided on the non-skin side of the back sheet 4. In FIG. 2, the body adhesive portions 51 and the wing adhesive portions 52 are illustrated by oblique lines, respectively.

In the present embodiment, the wing adhesive portions 52 are each formed by arranging two rectangle adhesives in the width direction, each of the adhesives having long sides in the longitudinal direction. Note that, the shape of each wing adhesive portion 52 is not particularly limited as long as the portion 52 has a size within the range of the size of the wing 100.

The body adhesive portions 51 are provided along the longitudinal direction at the inner side of the wing adhesive portions 52 in the width direction. Moreover, on the non-skin side of the back sheet 4, a front divided region 511 that divides the body adhesive portions 51 into front and back parts is provided in the front side of the wing adhesive portions 52 (in the front side of the discharge opening contact region 10A) in the longitudinal direction, and a back divided region 512 that divides the body adhesive portions 51 into front and back parts is provided in the back side of the wing adhesive portions 52 (in the back side of the discharge opening contact region 10A) in the longitudinal direction.

As stated above, the body adhesive portions 51 are divided into front and back parts by the front divided region 511 in the front side of the discharge opening contact region 10A, and the body adhesive portions 51 are divided into front and back parts by the back divided region 512 in the back side of the discharge opening contact region 10A. Accordingly, the napkin 1 can be raised toward the skin side of the wearer with the front divided region 511 and the back divided region 512 serving as starting points.

If the front divided region 511 and the back divided region 512 are not provided, a region crossing the body adhesive portions 51 serves as a starting point of raising the napkin 1. In this case, the napkin 1 is less likely to be raised to the skin side of the wearer due to the stiffness of the body adhesive portions 51. However, since the napkin 1 can be raised with the front divided region 511 and the back divided region 512 serving as starting points, the napkin 1 can easily maintain its flat shape in the front side and back side of the discharge opening contact region 10A.

In addition, the force deforming the napkin 1 into a protruded shape to the skin side by the bending guide portion 30 provided in the wing region 100A can be cut off by the front divided region 511 and the back divided region 512, and the guiding for the protruded deformation to the skin side by the bending guide portion 30 is eased in the front side and back side of the wing region 100A, thereby enabling the flat shape of the napkin 1 to be more easily maintained.

In the present embodiment, the front divided region 511 is provided on the front side (in the front side of the wing region 100A) of a front end 101 of each wing 100 in the longitudinal direction, and the back divided region 512 is provided in the back side (in the back side of the wing region 100A) of a back end 102 of each wing 100 in the longitudinal direction. As described above, the bending guide portion 30 is provided only in the wing region 100A.

Accordingly, in the wing region 100A that is a region larger in the longitudinal direction than the discharge opening contact region 10A, the deformation into a protruded shape to the skin side by the bending guide portion 30 can be maintained. In the front side and back side of the wing region 100A, the napkin 1 is raised toward the skin side of the wearer with the front divided region 511 and the back divided region 512 serving as starting points. Accordingly, the guiding for the protruded-deformation to the skin side by the bending guide portion 30 is eased, thereby enabling the flat shape to be easily maintained.

Note that, in the present embodiment, the napkin 1 includes the front divided region 511 and the back divided region 512. However, the divided region that divides the body adhesive portions 51 into front and back parts may be provided at least in the front side or back side of the wing adhesive portions 52 (in the front side or back side of the discharge opening contact region 10A) in the longitudinal direction.

As illustrated in FIG. 2, each body adhesive portion 51 is formed by arranging in the width direction four band-shaped adhesives disposed along the longitudinal direction respectively on both sides in the width direction of the bending guide portion 30. That is, the bending guide portion 30 and the body adhesive portions 51 do not have an overlapping portion in the width direction.

If the bending guide portion 30 and the body adhesive portions 51 include an overlapping portion in the width direction, when the napkin 1 is to be deformed into a protruded shape toward the skin side by the bending guide portion 30, a situation occurs in which portions of the body adhesive portions 51 which overlap the bending guide portion 30 in the width direction approach with one another in the width direction, and thus these portions are inadvertently bonded. However, since the bending guide portion 30 and the body adhesive portions 51 do not include the overlapping portion in the width direction, the body adhesive portions 51 are not bonded inadvertently when the napkin 1 is to be deformed into a protruded shape toward the skin side by the bending guide portion 30, thus easily and neatly forming the protruded shape to the skin side by the bending guide portion 30. The body adhesive portions 51 are not necessarily disposed on both sides in the width direction of the bending guide portion 30, and may be divided into front and back parts at least in the front side or the back side of the discharge opening contact region 10A.

Furthermore, as illustrated in FIG. 2, a front folding line 11 along the width direction is positioned in the front divided region 511, and similarly, a back folding line 12 along the width direction is positioned in the back divided region 512. The front folding line 11 and the back folding line 12 are folding lines for folding the napkin 1 in the longitudinal direction when the napkin 1 is wrapped.

The front folding line 11 and the back folding line 12 are each linearly formed from one end to another end of the napkin 1 in the width direction. Since the napkin 1 has a thickness, the front folding line 11 and the back folding line 12 each include a predetermined distance in front and back, while the front folding line 11 uses the reference line 11C as a center in the longitudinal direction, and the back folding line 12 uses the reference line 12C as a center in the longitudinal direction. In FIG. 2, the reference line 11C and the reference line 12C are illustrated by single-dotted chain lines, and a front end and back end of the front folding line 11 and a front end and back end of the back folding line 12 are illustrated by broken lines, respectively.

If the front folding line 11 and the back folding line 12 are provided at positions overlapping the body adhesive portions 51, when the napkin 1 is folded in the longitudinal direction at the time of wrapping, there is a possibility that wrinkles and distortion may occur in the back sheet 4 due to adhesiveness of the body adhesive portions 51. However, the front folding line 11 is disposed in the front divided region 511 where the adhesive is not present, and also the back folding line 12 is disposed in the back divided region 512 where the adhesive is not present, thereby preventing the occurrence of inadvertent wrinkles and distortion in the back sheet 4.

In addition, the protruded deformation to the skin side by the bending guide portion 30 along the longitudinal direction can be cut off by the front folding line 11 and the back folding line 12 along the width direction. Accordingly, the flat shape can be easily maintained in the front side of the front divided region 511 and in the back side of the back divided region 512.

As illustrated in FIG. 1 and FIG. 3, the top sheet 2 and the absorbent body 3 are integrated in a state of being joined in the thickness direction by the compressed portions 6. The compressed portions 6 are divided into central compressed portions 60, a front compressed portion 61 and a back compressed portion 62, and the entire shape thereof is in an annular shape which is elongated in the longitudinal direction along an outer edge of the absorbent body 3. A specific configuration and a positional relationship of the compressed portions 6 will be described later.

Moreover, the top sheet 2 and the absorbent body 3 are integrated in a state of being joined in the thickness direction by a crossing compressed portion 8 other than the compressed portions 6 (the central compressed portion 60, the front compressed portion 61, and the back compressed portion 62). The crossing compressed portion 8 is provided so as to cross the center portion in the width direction in the back side of the wing adhesive portions 52 (in the back side of the discharge opening contact region 10A) in the longitudinal direction. Note that, in the present embodiment, the crossing compressed portion 8 is disposed in the back sides of the wing region 100A.

Consequently, in the back side of the discharge opening contact region 10A, the force deforming the napkin 1 into a protruded shape to the skin side by the bending guide portion 30 provided along the longitudinal direction in the center portion in the width direction is less likely to be imparted, thereby enabling the flat shape to be more easily maintained.

Note that, in the compressed portions 6 and the crossing compressed portion 8, the thicknesses of the top sheet 2 and the absorbent body 3 decrease by applying pressure to the top sheet 2 and the absorbent body 3 in the thickness direction, and thus the density is higher than the surroundings.

### <Use aspect and shape when worn of napkin 1>

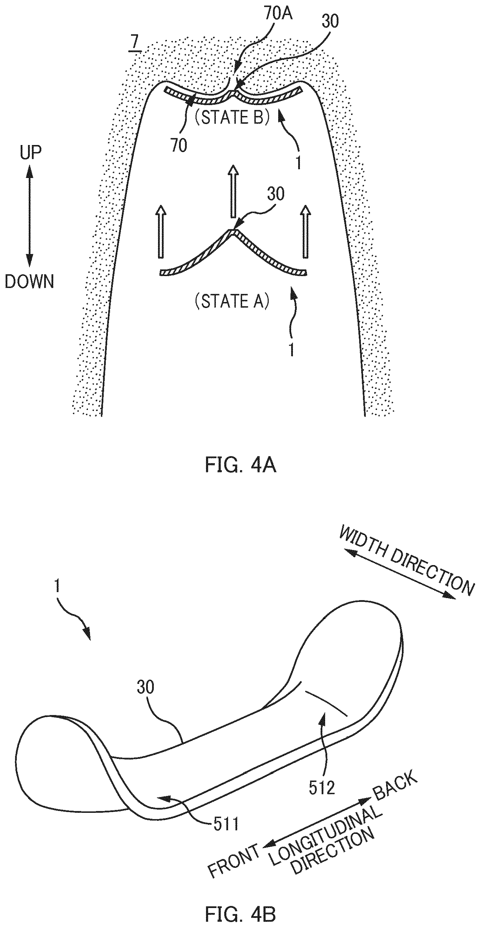

Next, a use aspect of the napkin 1, and a shape of the napkin 1 when being worn are described with reference to FIG. 4A and FIG. 4B.

FIG. 4A is a schematic cross-sectional view illustrating a use aspect of the napkin 1 and a state of the napkin 1 when being worn. FIG. 4B is a schematic perspective view illustrating an overall shape of the napkin 1 when being worn.

As illustrated in FIG. 4A, when a user 7 wears the napkin 1, the user 7 pulls up her undergarment or the like to a crotch 70 side of the user 7 while fixing the napkin 1 to the crotch portion of the undergarment or the like. At this time, as illustrated in a state A, the napkin 1 is deformed in a protruded shape to be raised in a state of being bent so that the center portion in the width direction protrudes to the skin side (upper side in FIG. 4A) at least in the discharge opening contact region 10A.

When the napkin 1 is applied to the crotch 70 of the user 7, the bending guide portion 30 comes into contact with the vaginal lips where a discharge opening 70A such as the vaginal introitus exists as illustrated in the state B. Accordingly, it becomes possible to make the user 7 feel an excellent fit of the napkin 1 and to allow the absorbent body 3 to absorb an excreted fluid such as the menstrual blood without leaking it to the outside.

As illustrated in FIG. 4B, the napkin 1 is raised toward the skin side of the wearer, with the front divided region 511 positioned in the front side of the discharge opening contact region 10A and the back divided region 512 positioned in the back side of the discharge opening contact region 10A serving as starting points. Thus, the guiding for the protruded deformation toward the skin side by the bending guide portion 30 is eased in the front side and back side of the discharge opening contact region 10A, thereby allowing the flat shape to be easily maintained. Accordingly, the napkin 1 is formed into a boat shape in appearance. Thus, the napkin 1 is easily in flat contact with the skin of the wearer in the front side (front side of the wearer) and the back side (buttocks side of the wearer) of the discharge opening contact region 10A, thereby improving a fitting property.

### <Configuration and positional relationship of compressed portion 6>

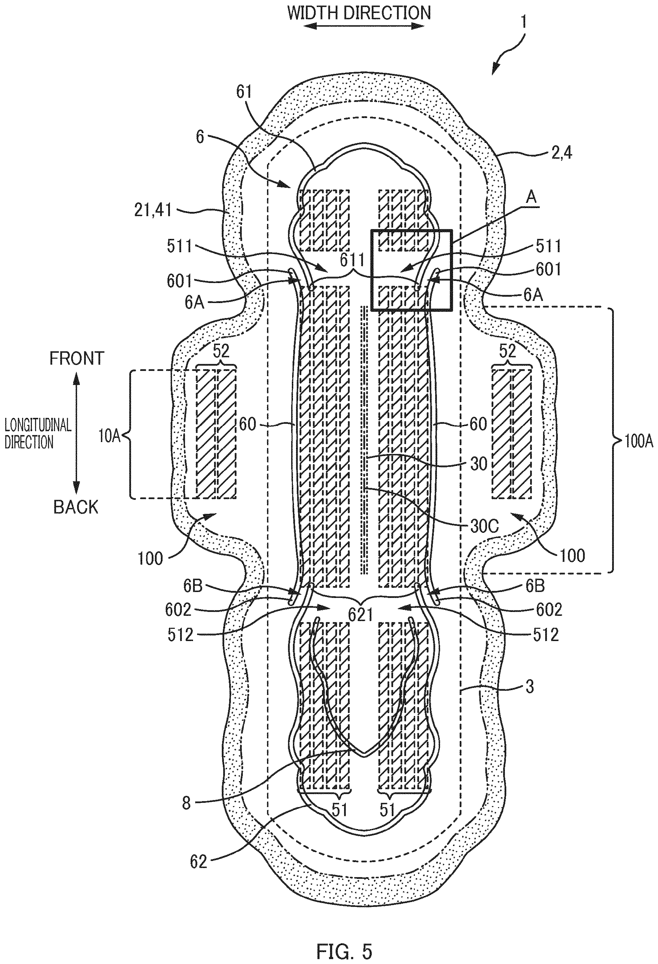

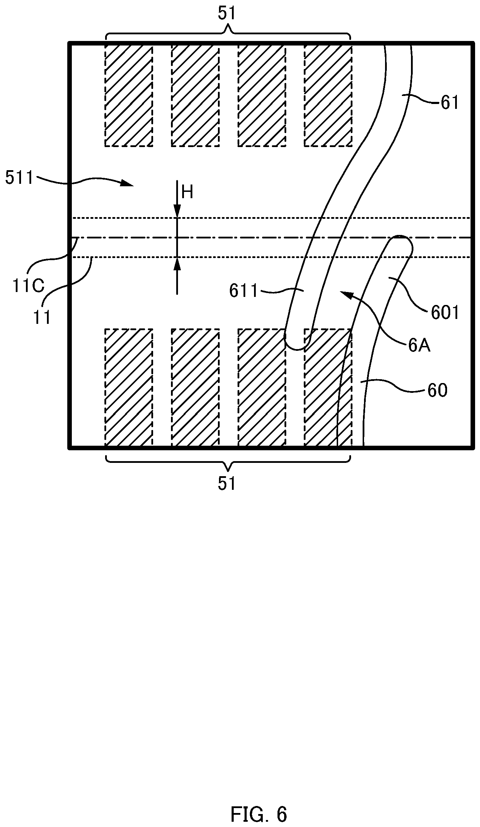

Next, a specific configuration and a positional relationship of the compressed portions 6 will be described with reference to FIG. 5 and FIG. 6.

FIG. 5 is an explanatory view illustrating a configuration and a positional relationship of the compressed portions 6. FIG. 6 is an enlarged view of an A-portion of FIG. 5. In FIG. 5, the body adhesive portions 51 and the wing adhesive portions 52 are shown by broken lines and oblique lines, respectively.

As illustrated in FIG. 5, the compressed portions 6 are divided into the central compressed portions 60 and the front compressed portion 61 by the front divided region 511, and are also divided into the central compressed portions 60 and the back compressed portion 62 by the back divided region 512. In this way, the compressed portions 6 are also divided into front and back parts together with the body adhesive portions 51 in the front divided region 511 positioned in the front side of the discharge opening contact region 10A and in the back divided region 512 positioned in the back side of the discharge opening contact region 10A, respectively. Accordingly, the guiding for the protruded deformation by the bending guide portion 30 is further eased in the front side and back side of the discharge opening contact region 10A, thereby allowing the flat shape to be easily maintained.

The central compressed portions 60 are substantially linearly provided along the longitudinal direction on both sides of the bending guide portion 30 in the width direction. In the present embodiment, the front compressed portion 61 is provided in the front side of the wing region 100A (see FIG. 2), and the back compressed portion 62 is provided in the back side of the wing region 100A. The front compressed portion 61 and the back compressed portion 62 (front and back compressed portions) each have a substantially arc shape.

As illustrated in FIG. 5 and FIG. 6, the central compressed portions 60 include tip portions 601 on the front divided region 511 side and tip portions 602 on the back divided region 512 side, the tip portions 601 and the tip portions 602 being respectively tilted from the inside to the outside in the width direction. Accordingly, the flat region sandwiched between a pair of central compressed portions 60 in the width direction is widened in the width direction from the center side toward the front side and from the center side toward the back side in the longitudinal direction of the napkin 1, and thus the napkin 1 can be in flat contact with the skin of the wearer in wider areas in the front side and back side of the napkin 1.

Moreover, the force deforming the napkin 1 into a protruded shape due to the bending guide portion 30 escapes toward the outside in the width direction along the central compressed portions 60, and thus the napkin 1 is more easily formed into a flat shape in the front side and back side of the discharge opening contact region 10A.

As illustrated in FIG. 5, each of the tip portions 601 on the front divided region 511 side of the central compressed portion 60 includes an overlapping portion overlapping with a tip portion 611 on the front divided region 511 side of the front compressed portion 61 in the longitudinal direction. Each of the tip portions 602 which are a back side group and are on the region 512 side of the central compressed portion 60 includes an overlapping portion overlapping with a tip portion 621 on the back divided region 512 side of the back compressed portion 62 in the longitudinal direction.

Accordingly, a front flat region 6A is formed between the tip portion 601 on the front divided region 511 side of the central compressed portion 60 and the tip portion 611 on the front divided region 511 side of the front compressed portion 61. Also, a back flat region 6B is formed between the tip portion 602 on the back divided region 512 side of the central compressed portion 60 and the tip portion 621 on the back divided region 512 side of the back compressed portion 62.

Since each of the front flat region 6A and the back flat region 6B is a region sandwiched between the compressed portions 6 having high stiffness, wrinkles and distortion are less likely to occur in the front flat region 6A and the back flat region 6B, thereby enabling the flat shape to be maintained. This can guide the raising of the napkin 1 toward the skin side of the wearer in a more flat manner in the front and back sides of the discharge opening contact region 10A.

Moreover, in the present embodiment, the tip portion 611 of the front compressed portion 61 is positioned at the inner side in the width direction with respect to the tip portion 601 on the front divided region 511 side of the central compressed portion 60, and the tip portion 621 of the back compressed portion 62 is positioned at the inner side in the width direction with respect to the tip portion 602 on the back divided region 512 side of the central compressed portion 60, respectively. For this reason, the force deforming the napkin 1 into a protruded shape by the bending guide portion 30 more easily escapes toward the outside in the width direction, thus more easily making the diaper 1 in the flat shape in the front and back sides of the discharge opening contact region 10A.

As illustrated in FIG. 6, the front folding line 11 is positioned at the center in the longitudinal direction of the front divided region 511. The tip of the tip portion 601 on the front divided region 511 side of the central compressed portion 60 is positioned at a reference line 11C of the front folding line 11. The tip portion 611 on the front divided region 511 side of the front compressed portion 61 is positioned on the wing region 100A side beyond the front folding line 11.

Accordingly, the front flat region 6A includes a non-overlapping portion that does not overlap the front folding line 11, and thus the front folding line 11 does not cross the front flat region 6A in the non-overlapping portion. This can maintain the flat shape of the front flat region 6A. Consequently, the raising of the napkin 1 toward the skin side of the wearer can be guided in a more flat manner in the front side of the discharge opening contact region 10A.

Depending on a dimension of a width H (see FIG. 6) in the longitudinal direction of the front folding line 11, there is a case that the region 6A between the tip portion 601 on the front divided region 511 side of the central compressed portion 60 and the tip portion 611 on the front divided region 511 side of the front compressed portion 61 does not completely overlap the front folding line 11, and this case is more desirable.

The relationship between the front folding line 11, and the central compressed portions 60 and the front compressed portion 61 has been described with reference to FIG. 6. This also applies to the relationship between the back folding line 12, and the central compressed portions 60 and the back compressed portion 62.

In the present embodiment, the compressed portions 6 have been divided into front and back parts in the front divided region 511 and the back divided region 512, respectively. However, the compressed portions 6 may be divided, for example, in either one of the front side or the back side, and also the compressed portions 6 may not be divided into front and back parts as long as at least the body adhesive portions 51 are divided into front and back parts in the front side or the back side of the discharge opening contact region 10A.

=== Others === The foregoing embodiments are intended to facilitate the understanding of the present disclosure but not to limit the present disclosure. And it is needless to say that modifications and improvements of the present disclosure are possible without departing from the scope of the disclosure, and equivalents thereof are also encompassed by the present disclosure.

In the foregoing embodiment, the napkin 1 includes both the front divided region 511 and the back divided region 512. However, the napkin 1 does not necessarily include the divided regions in both the front side and the back side, and may include a divided region at least in either one of the front side and back side.

### [Reference Signs List]

1··· napkin (absorbent article)
2···top sheet (skin side sheet)
3···absorbent body
4···back sheet (non-skin side sheet)
6···Compressed portion
11···front folding line
12···back folding line
30···bending guide portion
51···body adhesive portion
52···wing adhesive portion
60···central compressed portion
61···front compressed portion
62···back compressed portion
8···crossing compressed portion
100···wing
511···front divided region
512···back divided region
601, 602, 611, 621···tip portion

## Claims

1. An absorbent article including a longitudinal direction, a width direction and a thickness direction, the absorbent article comprising:
an absorbent body that absorbs liquid;
a skin side sheet disposed on a skin side of the absorbent body in the thickness direction;
a non-skin side sheet disposed on a non-skin side of the absorbent body in the thickness direction;
a pair of wings provided at a center portion in the longitudinal direction and projecting outwardly in the width direction;
a wing adhesive portion provided on the non-skin side of each of the wings; and
a body adhesive portion provided on the non-skin side of the non-skin side sheet along the longitudinal direction, the body adhesive portion being different from the wing adhesive portion;
a bending guide portion that guides the absorbent body to be bent to the skin side being provided along the longitudinal direction at a center portion in the width direction,
the bending guide portion including an overlapping portion overlapping with the wing adhesive portion in the longitudinal direction,
a divided region that divides the body adhesive portion into front and back being provided in a front side or back side of the wing adhesive portion in the longitudinal direction.

2. The absorbent article according to claim 1, wherein
the wings are provided in a region in the longitudinal direction between a position where a length in the width direction is the shortest in the front side of the wing adhesive portion and a position where a length in the width direction is the shortest in the back side of the wing adhesive portion, and
the divided region is provided in a front side of a front end of each of the wings or in a back side of a back end of each of the wings in the longitudinal direction.

3. The absorbent article according to claim 2, wherein
the divided region is provided in the front side of the front end of each of the wings and is also provided in the back side of the back end of each of the wings in the longitudinal direction.

4. The absorbent article according to any one of claims 1 to 3, wherein
a folding line along the width direction is positioned in the divided region.

5. The absorbent article according to any one of claims 1 to 4, wherein
the bending guide portion and the body adhesive portion do not include an overlapping portion in the width direction.

6. The absorbent article according to any one of claims 1 to 5, wherein
compressed portions along the longitudinal direction are provided on both sides in the width direction of the bending guide portion, and
the compressed portions are divided into front and back in the divided region.

7. The absorbent article according to claim 6, wherein
tip portions at a side of the divided region of central compressed portions positioned on a side of the center portion in the longitudinal direction in the compressed portions are tilted from an inside to an outside in the width direction.

8. The absorbent article according to claim 7, wherein
the tip portions at the side of the divided region of the central compressed portions each include, in the longitudinal direction, an overlapping portion overlapping with a tip portion at a side of the divided region of a front or back compressed portion positioned in a front side or back side of the central compressed portions in the longitudinal direction.

9. The absorbent article according to claim 8, wherein
a folding line along the width direction is positioned at a center in the longitudinal direction of the divided region, and
a region interposed between each of the tip portions of the central compressed portions and the tip portion of the front or back compressed portion include a non-overlapping portion that does not overlap with the folding line.

10. The absorbent article according to claim 8 or 9, wherein
the tip portion of the front or back compressed portion is positioned inwardly in the width direction with respect to each of the tip portions of the central compressed portions.

11. The absorbent article according to any one of claims 1 to 10, wherein
a crossing compressed portion that crosses the center portion in the width direction is provided in a back side of the wing adhesive portion in the longitudinal direction.

12. The absorbent article according to any one of claims 1 to 11, wherein
the bending guide portion is provided at a position overlapping with the wings in the longitudinal direction.
